# EUROPÄISCHE PATENTANMELDUNG

(11) **EP 1 714 615 A1**
(43) Veröffentlichungstag der Anmeldung: **25.10.2006**
(21) Anmeldenummer: 05008676.8
(22) Anmeldetag: 20.04.2005
(51) Int. Cl.: A61B 5/22

(54) **Magnetresonanztaugliches Ergometer**

(71) Anmelder: Schocke, Michael Dr., 6020 Innsbruck (AT); Greiner, Andreas Dr., 6063 Rum (AT)
(72) Erfinder: Schocke, Michael Dr., 6020 Innsbruck (AT); Greiner, Andreas Dr., 6063 Rum (AT)
(74) Vertreter: Vossius & Partner

(57) **Zusammenfassung**

Die vorliegende Erfindung beschreibt ein Ergometer, das insbesondere für die Untersuchung eines Probanden oder Patienten in einem Magnetresonanzgerät geeignet ist. Es weist dabei mindestens eine Testvorrichtung, welche im Wesentlichen beweglich angeordnet ist und mindestens eine pneumatische Kolben-Zylinderanordnung (7) auf. Die Testvorrichtung ist dabei an dem Kolben (20) oder dem Zylinder (21) angeordnet und eine pneumatische Druckbeaufschlagung zwischen Kolben (20) und Zylinder (21) stellt einen Widerstand bzw. eine Kraft für die Betätigung der Testvorrichtung durch den Probanden oder Patienten dar. Der Widerstand bzw. die Kraft zur Bewegung der Testvorrichtung, insbesondere während des Betriebs, ist steuerbar bzw. regelbar und der Kolben (20) und der Zylinder (21) sind so dimensioniert, dass sich zumindest beim Betrieb bzw. der Relativbewegung zwischen ihnen ein Luftkissen bildet. Der Widerstand bzw. die Kraft zur Betätigung der Testvorrichtung wird automatisch bzw. elektronisch basierend auf Messsignalen, die bei der Untersuchung eines Probanden oder Patienten rückgekoppelt werden, geregelt bzw. gesteuert.

## Beschreibung

Die vorliegende Erfindung betrifft ein Ergometer und ein Verfahren zum Betreiben eines Ergometers, das insbesondere für die Untersuchung, eines Probanden oder Patienten in einem Magnetresonanzgerät (MR) geeignet ist. Solche Untersuchungen werden unter anderem in der medizinischen Diagnostik und in den Sportwissenschaften durchgeführt.

Die Belastung von diversen Muskelgruppen gehört in der Sport-, Herz- und Kreislaufmedizin zu einer häufig verwendeten Methode, die Muskelfunktion zu überprüfen. Ein geeignetes Überprüfungsverfahren ist das sogenannte 31P-MRS-Verfahren (Phosphorus-31 Nuclear Magnetic Resonance Spectroscopy), wobei die Durchblutung eines stimulierten Muskels lokal in einem Magnetresonanzgerät gemessen wird. So können Energiemetaboliten im Muskel, wie zum Beispiel Phosphokreatin, anorganisches Phosphat oder Adenosintriphosphat, quantitativ gemessen werden. Das 31P-MRS-Verfahren erlaubt auch die Aufnahme der Energiemetaboliten unter Belastung der Wadenmuskulatur. Bei einer bestimmten Muskelbelastung kommt es anfänglich zu einem Abfall des Phosphokreatin, bis die Sauerstoffversorgung des Muskels soweit angehoben wird, dass der oxidative Metabolismus den erhöhten Energiebedarf allein bewältigen kann. In diesem Fall kommt es zu einem Gleichgewichtzustand des Phosphokreatin Zerfalles. Wenn aufgrund einer Stenose in einem versorgenden Gefäß die Durchblutung und somit die Sauerstoffversorgung nicht entsprechend dem Bedarf erhöht werden kann, fällt das Phosphokreatin progredient ab.

Der Gebrauch eines Ergometers in einem starken Magnetfeld ist problematisch, da viele Bauteile aus nicht-ferromagnetischen Metallen bzw. aus Holz oder Kunststoffen gefertigt werden müssen, um das Gerät, z. B. in einem Magnetresonanz-Tomographen, artefaktfrei und mit korrekter Funktion betreiben zu können. Auch Sensoren und Kabel zur Messung von Geräteparametern wie Kraft und Weg müssen so ausgelegt werden, dass sie keine Messartefakte verursachen. Des weiteren muss bei einem Ergometer eine definierte und reproduzierbare Kraft bzw. ein Widerstand eingestellt werden können, der für die Betätigung einer Testvorrichtung, die z.B. ein Pedal sein kann, durch den Probanden oder Patienten aufzubringen ist. Außerdem ist es sinnvoll, dass bei der Ergometrie die Arbeitsleistung während der Betätigung des Ergometers eingestellt, beziffert bzw. berechnet und entsprechend angepasst werden kann. Bei einem Ergometer drückt der Proband oder Patient mit einer festgelegten Frequenz gegen einen definierten Widerstand, wobei hier auch die Winkelauslenkung des Pedals und die Reibung eine große Rolle spielen. Diese Frequenz wird üblicherweise durch ein Metronom vorgegeben und die Kraft über Piezo-Technik gemessen. Bei handelsüblichen Ergometern, die außerhalb eines Magnetfeldes betrieben werden, wird die Krafteinstellung während der Übung automatisch durch entsprechende Elektronik oder Mechanik über einen Regelkreis nachreguliert, wenn der Proband die Frequenz oder die Winkelauslenkung nicht einhält. Nur durch solche Regelkreise kann ein Ergometer sinnvoll eingesetzt werden. Die derzeit handelsüblichen, MR-tauglichen Ergometer weisen einen solchen Regelkreis nicht auf. Darüber hinaus wird das Messergebnis bei den derzeit handelsüblichen Ergometern durch einen Reibungswiderstand bei der Betätigung der Testvorrichtung nicht-linear verfälscht, wodurch ein Messfehler nicht zuverlässig herausgerechnet werden kann.

Der Erfindung liegt daher die Aufgabe zugrunde, ein verbessertes Ergometer bzw. eine verbesserte Ergometeranordnung bereitzustellen, die die oben angeführten Nachteile vorzugsweise überwindet.

Die Aufgabe wird insbesondere durch die in den Patentansprüchen enthaltenen Merkmale gelöst.

Die Erfindung basiert auf dem Grundgedanken, ein Ergometer bereitzustellen, das insbesondere für die Untersuchung eines Probanden oder Patienten in einem Magnetresonanzgerät geeignet ist. Dabei muss vom Probanden oder Patienten an einer Testvorrichtung eine einstellbare Kraft aufgewendet werden, um eine Kolben-Zylinderanordnung in Bewegung zu setzen. Dies führt im Wesentlichen zu einer Relativbewegung zwischen Kolben und Zylinder. Die aufzubringende Kraft, um den durch die Kolben-Zylinderanordnung dargestellten Widerstand zu überwinden, kann insbesondere während der Betätigung der Testvorrichtung gesteuert bzw. geregelt werden. Dies kann manuell durch Fachpersonal oder auch durch entsprechende Anleitung, z.B. durch ein instruierendes Computerprogramm durchgeführt werden, wobei die Instruktionen im Wesentlichen aufgrund der ausgewerteten Messdaten entstehen. Ein solches Computerprogramm übernimmt vorzugsweise automatisch die Steuerung auch während des Betriebs. Vorzugsweise wird der Kraftbereich manuell vom Fachpersonal verändert und in dem jeweiligen Bereich dann automatisch geregelt. Die Auswertung der durch den Probanden erbrachten Arbeit bzw. Leistung ist sehr genau, da in der gegenwärtigen Erfindung die Reibungsverluste auf einen vernachlässigbar kleinen Wert minimiert wurden. Dadurch, dass in der Kolben-Zylinderanordnung zumindest bei einer Relativbewegung zwischen Kolben und Zylinder ein Polster, vorzugsweise ein Luftpolster, ausgebildet wird, können Reibungsverluste im Wesentlichen verhindert werden.

Alle verwendeten Bauteile wurden hinsichtlich Ihrer MRtauglichkeit geprüft, wobei das Ergometer vorzugsweise ausschließlich aus nicht-ferromagnetischen Materialien, wie z.B. Kunststoffe oder Holz, besteht und weiter ausschließlich mit Werkzeugen bearbeitet wurde, welche keine Metallabreibung an den einzelnen Bauteilen erzeugen.

Der Widerstand bzw. die Kraft, die an der Testvorrichtung zur Bewegung überwunden bzw. aufgebracht werden muss, wird durch ein pneumatisches System im Sinne eines Kolben-Zylindersystems erzeugt. Neben der bevorzugten Druckluft können Fluide aller Art eingesetzt werden. Vorzugsweise werden komprimierbare Fluide verwendet. Der Kolben ist dabei so konstruiert, dass sich zwischen Kolben und Zylinder ein schmales Luftkissen ausbildet, wodurch die Reibung soweit minimiert wird, dass sie für die Berechnung der geleisteten Arbeit vernachlässigbar ist. Zwischen einem Luftdruckanschluss und dem Zylinder ist ein Windkessel zwischengeschaltet, wodurch der Luftdruck stufenlos zu jeder Zeit während der Messung gesteuert oder geregelt werden kann. Dafür wird vorzugsweise ein Computergesteuertes Regelsystem verwendet, welches außerhalb des Faradayschen Käfig bildenden MR Geräts bedient wird. Somit befindet sich kein elektronisches Gerät in oder in der Nähe der MR-Röhre, um die Messergebnisse nicht zu beeinflussen. Das Regelmodul besteht dabei aus einem Drucksensor und einem Druckregler sowie einem Computer mit entsprechender Software.

Die Leistung der arbeitenden Muskulatur kann insbesondere durch den Druck im Zylinder, die Eintauchtiefe des Kolbens in den Zylinder und die Frequenz der Plantarflexion geregelt werden. Die tatsächliche Eintauchtiefe des Kolbens in den Zylinder wird gemessen, worauf in der Beschreibung der Ausführungsformen noch genauer eingegangen wird. Die Frequenz wird durch ein Metronom, dass unterschiedliche Frequenzen zur Verfügung stellt, vorgegeben, wobei der Proband oder Patient dem vorgegebenen Takt des Metronoms bei der Betätigung des Ergometers folgen sollte. Neben der Frequenz und dem Luftdruck ist auch die Eintauchtiefe des Kolbens in den Zylinder vorzugsweise stufenlos einstellbar. Ein entsprechendes Programm berechnet dabei die jeweiligen Parameter bei einer gewünschten Leistung in Watt oder die Wattzahl bei vorgegebenen Parametern.

In einer weiter bevorzugten Ausführungsform des Ergometers als Pedalergometer liegt der Proband oder Patient während der Messung vorzugsweise auf dem Rücken, wobei dessen Fuß mit zwei Riemen an einem Pedal fixiert ist. Zusätzlich bestehen weitere Riemenfixierungen im Bereich des Unterschenkels und der Schultern, um Bewegungsartefakte zu verhindern. Die Fixierung des Oberkörpers kann an die Körpergröße des Patienten angepasst werden, so dass die Plantarflexion jeweils bis zum vorgegeben Anschlag und in voller Streckung des Kniegelenks ausgeführt werden kann. Direkt unter dem zu messenden Muskel bzw. der zu messenden Muskelgruppe, vorzugsweise unter der Wadenmuskulatur wird eine Messspule platziert. Um das Messergebnis zu optimieren, wird die Wade in die Mitte der Messspule gelegt um so den Weg zwischen Wade und Messspule zu minimieren. Vorzugsweise wird nur eine Wade gemessen, um die Intensität des Messsignals im Zentrum unter der Wade zu nutzen und somit die Messzeit zu verkürzen. Dies ist weiterhin vorteilhaft, da Messartefakte von einer zweiten Wade das Ergebnis nicht beeinträchtigen.

Die Erfindung wird nachstehend anhand bevorzugter Ausführungsformen beispielhaft unter Bezugnahme auf die Zeichnungen beschrieben. Es zeigen:
- Fig. 1: eine schematische Ansicht einer Ausführungsform der erfindungsgemäßen Vorrichtung,
- Fig. 2a: eine schematische Ansicht einer Ausführungsform der Kolben-Zylinderanordnung der erfindungsgemäßen Vorrichtung,
- Fig. 2b: eine schematische Schnittansicht einer Ausführungsform der Kolben-Zylinderanordnung der erfindungsgemäßen Vorrichtung und
- Fig. 3: eine schematische Ansicht einer Ausführungsform der Wegmessung mittels Drehwinkelrad und Biegebalken der erfindungsgemäßen Vorrichtung.

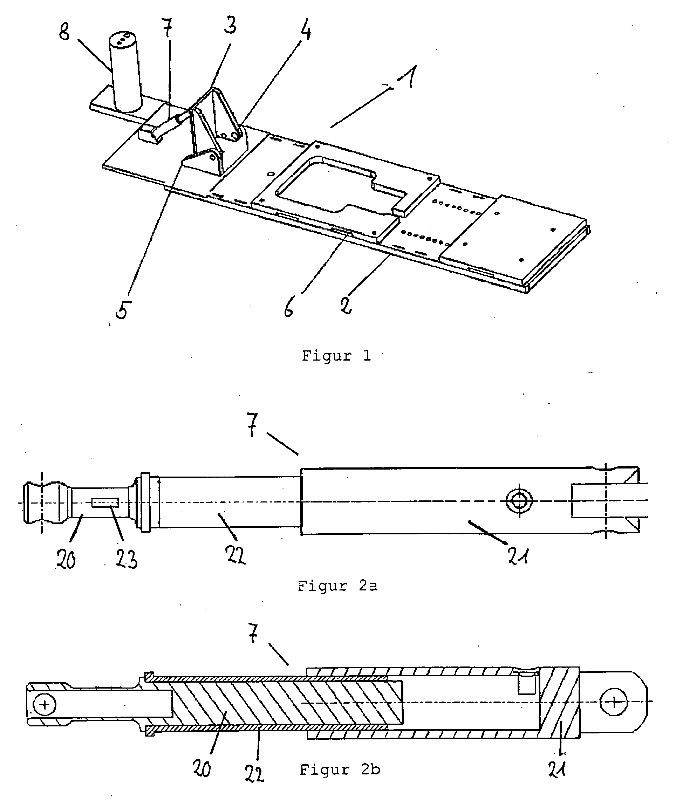

Figur 1 zeigt eine schematische, perspektivische Darstellung einer bevorzugten Ausführungsform eines Ergometers als Pedalergometer 1. Dabei bestehen die Bauteile des Ergometers 1 aus nicht-ferromagnetischen Materialien, um einen Einsatz in MR-Geräten zu ermöglichen. Auf einer Bodenplatte 2 ist eine Testvorrichtung, in dieser bevorzugten Ausführungsform ein Pedal 3, die im Wesentlichen beweglich über einen oder ein Paar Lagerbolzen 4 mit einer Halterung 5 angeordnet, wobei die Halterung 5 an der Bodenplatte 2 fest angebracht ist. Die Testvorrichtung variiert je nach dem zu messenden Körperteil bzw. zu belastenden Muskel bzw. Muskeln und ist vorzugsweise so gestaltet, dass bei Betätigung der Testvorrichtung im Wesentlichen der zu messende bzw. zu untersuchende Muskel belastet werden muss. So kann die Testvorrichtung so gestaltet sein, dass im Wesentlichen die gewünschten Muskeln bzw. Muskelgruppen durch bekannte Übungen, wie z.B. Butterfly, Bankdrücken, Biceps-Curl, Latissimuszug, Beinstrecken, Beinbeugen, Beinaduktion, Beinpresse und/oder Cable Cross, belastet werden. Andere Ausführungsformen der Testvorrichtung sind so z.B. Griffe, Schlaufen oder andere aus dem Kraftsport bekannte Vorrichtungen. Der zu messende Muskel bleibt dabei vorzugsweise auf der Messeinheit bzw. Messspule im Wesentlichen ruhig liegen. Das Pedal 3 wird deshalb aus dem Sprunggelenk betätigt, um die Wade möglichst ruhig über der Messspule zu halten. Das Pedal 3 ist vorzugsweise mittig zu einem zu messenden Körperteil eines Probanden oder Patienten anbringbar, damit die im Messspulenhalter 6 angeordnete Messspule möglichst gute Messergebnisse liefert. Eine pneumatische Kolben-Zylinderanordnung 7 ist zwischen dem Pedal 3 und einer Halterung an der Bodenplatte 2 so angeordnet, dass bei Betätigung des Pedals 3 durch einen Probanden oder Patienten der Kolben 20 relativ zum Zylinder 21 bewegt wird. Die pneumatische Druckbeaufschlagung der Kolben-Zylinderanordnung 7 stellt bei der Betätigung des Pedals 3 durch den Probanden oder Patienten einen Widerstand bzw. eine Kraft dar. Das Pedal 3 weist dabei vorzugsweise einen Anschlag auf, der den Kolben 20 davor schützt, am Boden des Zylinders 21 anzustoßen. Auf diese Weise kann eine Beschädigung der Kolben-Zylinderanordnung 7 vermieden werden.

Die pneumatische Druckbeaufschlagung der Koben-Zylinderanordnung 7 wird durch eine Luftdruckquelle, die am Zylinder 21 angeschlossen ist, erreicht. Zwischen Ergometer 1 und Luftdruckquelle ist vorzugsweise ein Windkessel 8 angeordnet. Dieser Windkessel 8 ist so ausgebildet, dass der an der Kolben-Zylinderanordnung 7 anliegende Luftdruck mit Hilfe einer Regelung, auf die im Folgenden eingegangen wird, steuerbar bzw. regelbar ist und gewährleistet darüber hinaus, dass sich bei Betätigung der Testvorrichtung bzw. des Pedals 3 durch den Gegendruck keine großen bzw. kurzzeitigen Belastungsspitzen aufbauen. Die aufzubringende Kraft liegt in einem Bereich von 50 N bis 900 N, bzw. von 100 N bis 800 N, bzw. von 200 N bis 700 N, bzw. von 300 N bis 600 N und beträgt vorzugsweise ca. 600 N bei gesunden Probanden und ca. 300 N bei in der entsprechenden Bewegung behinderten Patienten.

Das Ergometer 1 weist vorzugsweise eine Regeleinrichtung auf, deren Sensoren und zugehörige abgeschirmte Leitungen innerhalb des Untersuchungsraums des Magnetresonanzgerätes angeordnet sein können. Die Steuerelektronik der Regeleinrichtung befindet sich außerhalb des Untersuchungsraums des Magnetresonanzgerätes. Die Regeleinrichtung reguliert den Luftdruck an der Kolben-Zylinderanordnung 7 und damit den auf den Kolben 20 wirkenden Druck, der die aufzubringende Kraft, die zur Betätigung der Testvorrichtung bzw. des Pedals 3 durch einen Probanden oder Patienten nötig ist. Diese Regeleinrichtung weist vorzugsweise einen Computer und ein Computerprogramm zur Regelung und/oder Verarbeitung von akquirierten Daten auf.

Die Ermittlung der an dem Pedal 3 zu erbringenden Kraft erfolgt über einen oder mehrere Sensoren, die eine mechanische Deformation in ein elektrisches Signal oder in eine elektrische Widerstandsänderung umwandeln, die am Kolben 20 angeordnet sind. Vorzugsweise wird hier ein Dehnungsmessstreifen (DMS) 23 verwendet. Der Durchmesser des Kolbens 20 der Kolben-Zylinderanordnung 7 ist dabei im Bereich eines Dehnungsmessstreifens schlanker als in der Zylinder-Führung. Des weiteren kann zumindest im Bereich des Dehnungsmessstreifens der Kolben 20 hohl ausgeführt sein. In einer bevorzugten Ausführungsform liegt der Elastizitätsmodul des Kolbens 20 idealerweise bei 3000 N/mm².

Der Widerstand bzw. die Kraft zur Bewegung der Testvorrichtung bzw. des Pedals 3 ist insbesondere während des Betriebs steuerbar bzw. regelbar. Die Regelung des Widerstands bzw. der Kraft kann automatisch bzw. elektronisch, basierend auf den bei der Untersuchung eines Probanden oder Patienten gemessenen Signalen geregelt oder z.B. manuell gesteuert werden. Des weiteren sind Kolben 20 und der Zylinder 21 so dimensioniert, dass sich zumindest beim Betrieb bzw. der Relativbewegung zwischen ihnen ein Luftkissen bildet. Durch dieses Luftkissen wird der Reibungswiderstand vernachlässigbar gering und folglich die Messwerte erheblich genauer. Die Kolben-Zylinderanordnung 7 weist dabei zwischen dem Kolben 20 und dem Zylinder 21 einen Spalt von 0,02 mm bis 0,05 mm, bzw. 0,03 mm bis 0,05 mm, bzw. 0,02 mm bis 0,04 mm, bzw. 0,03 bis 0,04 mm und vorzugsweise einen Spalt von etwa 0,035 mm auf.

In einer bevorzugten Ausführungsform als Pedalergometer sind ferner Fixierungen, wie z.B. eine Riemenfixierung für den Fuß eines Probanden, seinen Unterschenkel und seine Schultern angebracht vorgesehen, die auch entsprechend der Körpergröße des Probanden oder Patienten einstellbar sind.

Um den Weg der Bewegung des Pedals 3 zu messen, wird eine Carbonfeder parallel zum Pneumatikzylinder 21 angeordnet, die mindestens einen Dehnungsmessstreifen aufweist. So lässt sich in Abhängigkeit des Ausgangssignals des Dehnungsmessstreifens auf die Pedalbewegung rückschließen. Diese Carbonfeder ist so gestaltet, dass sie über einen weiten Federweg eine Federkonstante aufweist und die Feder in diesem Bereich des Federwegs betätigt wird.

Figur 2a zeigt eine schematische Darstellung einer Kolben-Zylinderanordnung 7 und Figur 2b eine Schnittdarstellung dieser Kolben-Zylinderanordnung 7. Mittels eines Adapters 22 kann der durch den Probanden oder Patienten zu erbringende Kraftbereich, in Bereichen wie oben angegeben, verändert werden, vorzugsweise davon abhängig, ob ein Patient oder ein Proband untersucht wird. Dies wird erreicht, indem der im Zylinder 21 wirksame Kolbendurchmesser durch einen den Kolben 20 umschließenden Adapter 22 wahlweise an dem Zylinder 21 oder an dem Kolben 20 fest angebracht wird. Vorzugsweise wird der Adapter 22 mit dem jeweiligen Teil verschraubt.

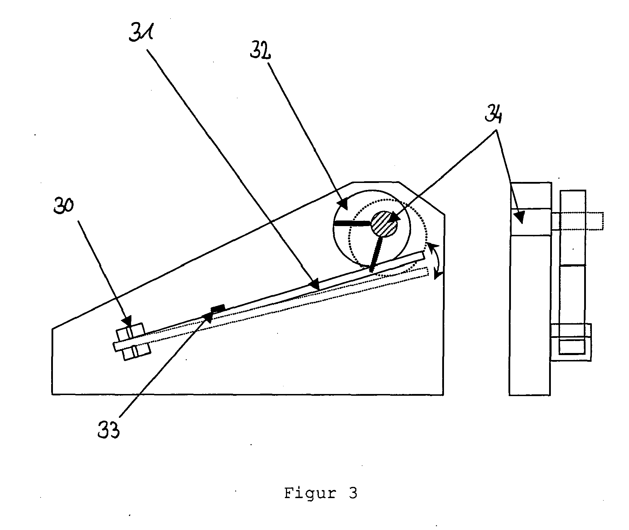

Figur 3 zeigt die Wegmessung mittels eines Winkelrades 32 und eines Biegebalkens 31. Dabei wird bei der Betätigung des Pedals 3 der Biegebalken 31 ausgelenkt, wobei der Weg des Pedals 3 ermittelt wird. Dabei wird das Drehwinkelrad 32 an einer als Achse eingesetztem exzentrischen Drehbolzen 34 angebracht, wobei das Drehwindelrad 32 in Folge der Betätigung der Testvorrichtung bzw. des Pedals 3 bewegt wird und so den Biegebalken 31 auslenkt. Der Biegebalken 31, der an einer Seite in einer Vorrichtung 30 fest angebracht ist, wird dadurch ausgelenkt. An dem Biegebalken 31 ist mindestens ein Dehnungsmessstreifen 33 so angeordnet, dass die Durchbiegung des Biegebalkens 31 in ein elektronisches Signal umgewandelt wird.

Die Erfindung umfasst ebenfalls Ausführungsformen, die Merkmale aus verschiedenen, vorstehend beschriebenen Ausführungsformen kombiniert.

## Patentansprüche

1. Ergometer, insbesondere für die Untersuchung eines Probanden oder Patienten in einem Magnetresonanzgerät, mit:
mindestens einer Testvorrichtung, welche im Wesentlichen beweglich angeordnet ist,
mindestens einer pneumatischen Kolben-Zylinderanordnung (7), wobei die Testvorrichtung zur Bewegung an dem Kolben (20) oder dem Zylinder (21) angeordnet ist und eine pneumatische Druckbeaufschlagung zwischen Kolben (20) und Zylinder (21) einen Widerstand bzw. eine Kraft für die Betätigung der Testvorrichtung durch den Probanden oder Patienten darstellt,
**dadurch gekennzeichnet, dass**
der Widerstand bzw. die Kraft zur Bewegung der Testvorrichtung, insbesondere während des Betriebs, steuerbar bzw. regelbar ist.

2. Ergometer, insbesondere für die Untersuchung eines Probanden oder Patienten in einem Magnetresonanzgerät, mit:
mindestens einer Testvorrichtung, welche im Wesentlichen beweglich angeordnet ist,
mindestens einer pneumatischen Kolben-Zylinderanordnung (7), wobei die Testvorrichtung zur Bewegung an dem Kolben (20) oder dem Zylinder (21) angeordnet ist und eine pneumatische Druckbeaufschlagung zwischen Kolben (20) und Zylinder (21) einen Widerstand bzw. eine Kraft für die Betätigung der Testvorrichtung durch den Probanden oder Patienten darstellt,
**dadurch gekennzeichnet, dass**
der Kolben (20) und der Zylinder (21) so dimensioniert sind, dass sich zumindest beim Betrieb bzw. der Relativbewegung zwischen ihnen ein Luftkissen bildet.

3. Ergometer nach Anspruch 1,
wobei der Widerstand bzw. die Kraft automatisch bzw. elektronisch basierend auf Messsignalen, die bei der Untersuchung eines Probanden oder Patienten rückgekoppelt werden, geregelt bzw. gesteuert wird.

4. Ergometer nach Anspruch 1, 2 oder 3,
wobei die Testvorrichtung ein Pedal (3) ist.

5. Ergometer nach Anspruch 4, wobei das Ergometer ferner eine Riemenfixierungen für den Fuß eines Probanden oder Patienten, seinen Unterschenkel und seine Schultern aufweist.

6. Ergometer nach Anspruch 4 oder 5,
wobei die Riemenfixierung für die Schultern der Körpergröße des Probanden entsprechend einstellbar ist.

7. Ergometer nach einem der vorstehenden Ansprüche,
wobei die Kolben-Zylinderanordnung (7) zwischen dem Kolben (20) und dem Zylinder (21) einen Spalt von mindestens 0,02 mm und maximal 0,05 mm aufweist.

8. Ergometer nach einem der vorstehenden Ansprüche,
wobei die Bauteile des Ergometers aus nicht-ferromagnetischen Materialien bestehen.

9. Ergometer nach einem der vorstehenden Ansprüche,
wobei die Testvorrichtung einen Anschlag aufweist, der den Kolben (20) davor schützt, am Boden des Zylinders (21) anzustoßen.

10. Ergometer nach einem der vorstehenden Ansprüche, wobei die Ermittlung der an der Testvorrichtung zu erbringenden Kraft über die Messung des Luftdrucks im Zylinder (21) oder in der Druckluftzuführung erfolgt.

11. Ergometer nach einem der vorstehenden Ansprüche, wobei die Ermittlung der an der Testvorrichtung zu erbringenden Kraft über mindestens einen Dehnungsmessstreifen am Kolben erfolgt.

12. Ergometer nach Anspruch 11,
wobei der Kolbendurchmesser im Bereich des Dehnungsmessstreifens schlanker ist als in der Zylinder-Führung.

13. Ergometer nach Anspruch 11,
wobei der Kolben (20) zumindest im Bereich des Dehnungsmessstreifens hohl ausgeführt ist.

14. Ergometer nach einem der vorstehenden Ansprüche, wobei der Elastizitätsmodul des Kolbens (20) idealerweise bei 3000 N/mm² liegt.

15. Ergometer nach einem der vorstehenden Ansprüche, wobei die Wegmessung der Bewegung der Testvorrichtung über eine parallel zum Pneumatikzylinder angeordnete Carbonfeder, die mindestens einen Dehnungsmessstreifen aufweist, erfolgt.

16. Ergometer nach Anspruch 15, wobei die Carbonfeder so gestaltet ist, dass sie über einen weiten Federweg eine Federkonstante aufweist und die Feder in diesem Bereich betätigt wird.

17. Ergometer nach einem der vorstehenden Ansprüche, wobei die Wegmessung mittels eines Biegebalkens (31), der bei Betätigung der Testvorrichtung ausgelenkt wird, ermittelt wird.

18. Ergometer nach Anspruch 17, wobei ein Drehwinkelrad (32) an einer als Achse eingesetztem exzentrischen Drehbolzen (34) fest angebracht und so angeordnet ist, dass es in Folge der Betätigung der Testvorrichtung bewegt wird und so den Biegebalken (31) auslenkt.

19. Ergometer nach Anspruch 17 oder 18, wobei der Biegebalken (31) an einer Seite mittels einer Befestigungsvorrichtung (30) fest angebracht ist und in einem Abstand dazu ausgelenkt wird.

20. Ergometer nach Anspruch 17, 18 oder 19, wobei an dem Biegebalken (31) mindestens ein Dehnungsmessstreifen (33) so angeordnet ist, dass die Durchbiegung des Biegebalkens (31) in ein elektronisches Signal umgewandelt wird.

21. Ergometer nach einem der vorstehenden Ansprüche, wobei die Testvorrichtung mittig zu einem zu messenden Körperteil eines Probanden oder Patienten angebracht ist.

22. Ergometer nach einem der vorstehenden Ansprüche, wobei die durch den Probanden oder Patienten zu erbringende Kraft verändert wird, indem der im Zylinder (21) wirksame Kolbendurchmesser durch einen den Kolben (20) umschließenden Adapter (23), der wahlweise mit dem Zylinder (21) oder dem Kolben (20) verbunden ist, verändert wird.

23. Ergometer nach einem der vorstehenden Ansprüche, wobei die an der Testvorrichtung aufzubringende Kraft bis ca. 600 N bei gesunden Probanden und idealerweise ca. 300 N bei Patienten beträgt.

24. Ergometer-Anordnung mit einem Ergometer nach einem der vorstehenden Ansprüche,
wobei die Anordnung weiter eine Luftdruckquelle und einen Windkessel (8) aufweist, der zwischen Luftdruckquelle und Ergometer angeordnet ist.

25. Ergometer-Anordnung mit einem Ergometer nach Anspruch 24,
wobei der Windkessel (8) so ausgebildet ist, dass der an der Kolben-Zylinderanordnung (7) anliegende Luftdruck steuerbar bzw. regelbar ist und gewährleistet, dass sich bei Betätigung der Testvorrichtung durch den Gegendruck keine großen bzw. kurzzeitigen Belastungsspitzen aufbauen.

26. Ergometer-Anordnung nach einem der Ansprüche 24 oder 25,
wobei die Anordnung ferner eine Regeleinrichtung aufweist, deren Sensoren und zugehörige abgeschirmte Leitungen innerhalb des Untersuchungsraums des Magnetresonanzgerätes angeordnet sind und die Steuerelektronik der Regeleinrichtung sich außerhalb des Untersuchungsraums des Magnetresonanzgerätes befindet.

27. Ergometer-Anordnung nach Anspruch 26,
wobei die Regeleinrichtung den Luftdruck an der Kolben-Zylinderanordnung (7) und damit den auf den Kolben (20) wirkenden Druck reguliert, der die zu erbringende Kraft, die zur Betätigung der Testvorrichtung durch einen Probanden oder Patienten nötig ist, bestimmt.

28. Ergometer-Anordnung nach Anspruch 26 oder 27, wobei die Regeleinrichtung einen Computer und ein Computerprogramm zur Regelung und/oder Verarbeitung vom akquirierten Daten aufweist.

29. Verfahren zum Betreiben eines Ergometers,
insbesondere nach einem der Ansprüche 1, 7 oder 8, in einem Magnetresonanzgerät, mit den Schritten:
Bereitstellen mindestens einer bewegbaren Testvorrichtung,
Bereitstellen mindestens einer pneumatischen Kolben-Zylinderanordnung (7) mit Anordnen der Testvorrichtung an dem Kolben (20) oder dem Zylinder (21) und Verursachen eines Widerstandes bzw. einer Kraft für die Betätigung der Testvorrichtung durch den Probanden oder Patienten durch eine pneumatische Druckbeaufschlagung zwischen Kolben (20) und Zylinder (21),
**gekennzeichnet durch**
Steuern bzw. Regeln des Widerstandes bzw. der Kraft zur Bewegung der Testvorrichtung, insbesondere während des Betriebs.

30. Verfahren zum Betreiben eines Ergometers,
insbesondere nach einem der Ansprüche 2, 7 oder 8, in einem Magnetresonanzgerät, mit den Schritten:
Bereitstellen mindestens einer bewegbaren Testvorrichtung,
Bereitstellen mindestens einer pneumatischen Kolben-Zylinderanordnung (7) mit Anordnen der Testvorrichtung an dem Kolben (20) oder dem Zylinder (21) und Verursachen eines Widerstandes bzw. einer Kraft für die Betätigung der Testvorrichtung durch den Probanden oder Patienten durch eine pneumatische Druckbeaufschlagung zwischen Kolben (20) und Zylinder (21),
**gekennzeichnet durch**
Ausbilden eines Luftkissens zwischen Kolben (20) und Zylinder (21) beim Betrieb bzw. der Relativbewegung zwischen ihnen.

31. Verwendung eines Ergometers, einer Ergometer-Anordnung bzw. eines Verfahrens nach einem der vorstehenden entsprechenden Ansprüche für die medizinische Diagnostik und in den Sportwissenschaften, insbesondere für ein P NMR-Verfahren.
